# EUROPEAN PATENT APPLICATION

(11) **EP 2 301 504 A1**
(43) Date of publication of application: **30.03.2011**
(21) Application number: 09171124.2
(22) Date of filing: 23.09.2009
(51) Int. Cl.: A61F 13/66, A61F 13/74

(54) **Undergarment for use with an absorbent article**

(71) Applicant: Attends Healthcare AB, 578 24 Aneby (SE)
(72) Inventor: Svensson, Ida, 554 50, JÖNKÖPING (SE); Håkansson, Mikael, 573 37, TRANÅS (SE); Persson, Christer, 578 31, ANEBY (SE); Lindqvist, Arne, 152 57, SÖDERTÄLJE (SE)
(74) Representative: Jacobsson, Peter

(57) **Abstract**

The invention concerns an undergarment (1) for use with an absorbent article (5), wherein the undergarment is provided with at least one landing zone (2, 2') for cooperating with attachment means (6, 6') of the absorbent article, the at least one landing zone (2, 2') indicating placement of the attachment means of the absorbent article in the undergarment. The landing zone (2, 2') further comprises a material having an affinity for pressure sensitive adhesive for cooperating with attachment means of an absorbent article, which attachment means comprises pressure sensitive adhesive. The invention also concerns a kit of parts comprising an undergarment as stated above and a number of absorbent articles having attachment means comprising pressure sensitive adhesive.

## Description

### Technical field

The present document relates to an undergarment for use with an absorbent article. The document particularly relates to an undergarment for use with an incontinence pad.

### Background

When using an absorbent article, such as an incontinence or menstrual protection article, such as a sanitary or incontinence napkin, towel or pad, the article is usually placed either in the undergarment that the user normally uses or in a specially designed undergarment for use with the specific article.

To achieve an optimal protection against leakage it is important that the article is placed correctly in the undergarment and that the article can stay in place in the undergarment during use. Further, it has to be possible to release the article from the undergarment after use.

For this reason, there are undergarments, such as the undergarments described in GB1221002 and US4664663, which use hook-and-loop solutions for securely holding the absorbent article in position in the undergarment. In these solutions, the undergarment has a hook-like element arranged to cooperate with a loop-like element arranged on the absorbent article. Such a hook-and-loop solution is rather expensive and would therefore find limited use.

US5415650 shows an undergarment with a strip of cohesive-adhesive material impregnated to the undergarment. The strip is arranged to cooperate with a similar cohesive-adhesive strip arranged on an absorbent article, for keeping the absorbent article in place in the undergarment. A cohesive-adhesive material has an affinity for itself, i.e. it will only attach to materials similar to itself. Thereby, there is a low risk that the cohesive-adhesive strip will stick to other parts of the undergarment. This arrangement with cohesive-adhesive strips on both undergarment and absorbent articles will also be rather expensive and therefore find limited use.

Consequently, there is a need for an alternative undergarment that provides a cost-efficient solution for optimally protecting against leakage when an absorbent article, such as an absorbent pad, is placed in the undergarment. The undergarment should provide a cost-efficient solution for facilitating correct positioning of the absorbent article in the undergarment and for securing that the absorbent article stays in place in the undergarment during use. Further, it has to be possible to release the article from the undergarment after it has been used.

### Summary

In the prior art undergarments, not only the undergarment but also the absorbent articles have been adapted to achieve a solution in which an absorbent article can be correctly and securely placed in the undergarment, and in which the article can be easily removed from the undergarment after use. The problem is that such a combination of undergarment and absorbent article becomes rather expensive, not the least since the absorbent articles have to be adapted to function with the undergarment of the prior art. When using an undergarment with interchangeable absorbent articles, there are numerous absorbent articles used for each undergarment. Therefore, the cost of an absorbent article to be used together with an undergarment influences in a very high degree the total cost for a user of such a combination of undergarment and absorbent article. If regular absorbent articles, which are already cost-efficient, can be used together with a specially adapted undergarment, the total cost for a user of a combination of undergarment and absorbent article would be low. To achieve such a cost-efficient solution, the applicants have come to the conclusion that it would be appropriate to adapt the landing zone of an undergarment to the attachment zone of a regular absorbent article.

A regular absorbent article, e.g. incontinence pad, of today normally has an attachment means arranged on its back sheet, for arranging the absorbent article in the undergarment. The attachment means normally comprises a pressure sensitive adhesive. According to the invention, the undergarment is arranged with a landing zone indicating placement of the attachment means of the absorbent article in the undergarment, the landing zone having an affinity for pressure sensitive adhesive, for cooperating with attachment means of an absorbent article, which attachment means comprises pressure sensitive adhesive. Thereby, a cost-efficient solution is achieved, which at the same time can facilitate correct positioning of the absorbent article in the undergarment and secure that the absorbent article stays in place in the undergarment during use. The solution is cost-efficient since the undergarment can be used with regular absorbent articles having attachment means of a pressure sensitive adhesive. It is not necessary to make any modifications in existing absorbent articles. Also, it may be possible to use the undergarment of the invention with absorbent articles of many different providers, as long as the attachment means comprises pressure sensitive adhesive, and are positioned similarly in the different absorbent articles.

By the expression that the landing zone has an affinity for pressure sensitive adhesive is meant that the landing zone has an adherence to pressure sensitive adhesive. I.e. if the landing zone is joined to an area of pressure sensitive adhesive, there is an adhesive strength between the landing zone and the area of pressure sensitive adhesive. More specifically, a force higher than the adhesive strength is needed to take the landing zone and the area of pressure sensitive adhesive apart.

A pressure sensitive adhesive (PSA) is an adhesive that forms a bond with an adherend when pressure is applied to marry the adhesive with the adherend.

According to an embodiment of the invention, the landing zone of the undergarment only has an affinity for pressure sensitive adhesive. Thereby, the landing zone will not risk sticking onto other materials, such as other parts of the undergarment or other parts of the absorbent article, which in that case would cause trouble to the user when trying to attach the absorbent article to the undergarment.

According to another embodiment of the invention, the material of the landing zone is ink paste that has been applied to the undergarment in an area of the undergarment where the landing zone is supposed to be. By using a landing zone comprising ink paste applied to the undergarment, a landing zone that has a suitable affinity value for pressure sensitive adhesive is achieved. Also, a landing zone that is easily manufactured is achieved, resulting in a cost-efficient undergarment.

According to another embodiment, the landing zone is a piece of material that is arranged separately to the undergarment, e.g. by being sewn or glued to the undergarment. By arranging the landing zone as a piece of material arranged separately to the undergarment, it can have a different elasticity value than the undergarment, e.g. an elasticity value similar to the elasticity of the absorbent article. Thereby, the risk is low that an absorbent article will loosen from the undergarment if the undergarment is stretched.

According to yet another embodiment, after the landing zone and the attachment means of the absorbent article has been joined together, the adhesive strength between the landing zone and the attachment means of the absorbent article is 0.5 - 4 Newton, preferably 1.0 - 2.5 Newton, most preferably 1.5 - 2.0 Newton, measured on an adhesive width of 45 mm. Thereby, an adhesive strength necessary for keeping the absorbent article in place in the undergarment during use is achieved. Further, the adhesive strength is not too high for it to be convenient for a user to take an already used absorbent article out of the undergarment.

According to still another embodiment, the landing zone is coated with a release agent. Thereby, it can be easily controlled that the adhesive strength between the landing zone and the attachment means is limited to the said values in Newton.

According to another embodiment, the landing zone has an elasticity that is less than 30 % of the elasticity of a part of the undergarment arranged within a radius of 3 cm from the landing zone, preferably less than 20 %, more preferably less than 10 %, most preferably less than 5 %. Thereby, there is low risk that the absorbent article will loosen from the landing zone when the undergarment is stretched, for example when a user puts the undergarment with the absorbent article attached, onto the body. A normal absorbent article has low elasticity and an undergarment normally has much higher elasticity than the absorbent article, to be able to fit persons of different body sizes. If the landing zone would have the same elasticity as the crotch portion of the undergarment, the absorbent article will loosen from the undergarment, if the absorbent article was joined to the undergarment before the undergarment is stretched.

According to yet another embodiment, the position of the landing zone in the undergarment corresponds to the position of the attachment means of an absorbent article, such that if the landing zone is joined to the attachment means of an absorbent article, the article is correctly placed in the undergarment. Thereby, the undergarment indicates correct placement of many types and sizes of absorbent articles, which makes it easy for the user to correctly place the article in the undergarment.

According to still another embodiment, a surface area of the landing zone is arranged to be 10-30 % larger, preferably 15-25 % larger, than a corresponding surface area of the attachment means of an absorbent article to be used with the undergarment. By making an area of the landing zone a bit larger than an area of the attachment means, the areas being arranged to be joined to each other when the absorbent article is positioned in the undergarment, it will be easy for a user to put the attachment means onto the landing zone of the undergarment. I.e. the risk will be low that the absorbent article is partly positioned outside the undergarment, risking a too high or too low attachment force or that the undergarment may be torn when the absorbent article is released from the undergarment.

According to another aspect, the invention concerns a kit of parts comprising an undergarment according to the invention and at least one absorbent article provided with attachment means of a pressure sensitive adhesive for attaching the absorbent article to the undergarment.

### Brief Description of the Drawings

Embodiments of the present solution will now be described, by way of example, with reference to the accompanying schematic drawings.
Fig 1 is a schematic perspective view of an undergarment with landing zone and an absorbent article to be placed in the undergarment.
Fig. 2 is a schematic perspective view of another undergarment with landing zone and an absorbent article to be placed in the undergarment.

### Description of Embodiments

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

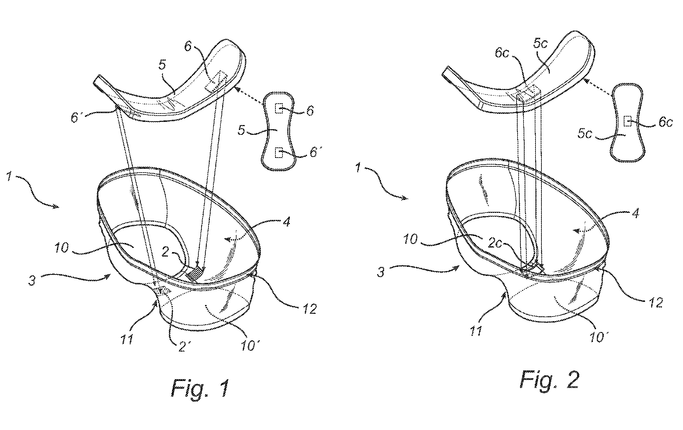

Figure 1 illustrates an undergarment 1 comprising a front part 3, a rear part 4, two openings 10, 10' for the legs of a user of the undergarment, and a crotch region 11, connecting the leg openings 10, 10' and the front 3 and rear 4 parts. Also, the undergarment has an upper part 12, defining a waist opening, for surrounding a user's waist when the undergarment is in place on a user's body. Further, when in place, an inside of the undergarment is intended to face a user's body. Further, the front part 3 is intended to face the front side of a user's body and the rear part 4 is intended to face the back side of the user's body.

The crotch region 11 of the undergarment may be designed such as to provide optimal comfort to the wearer, while still comprising a sufficient area to place an absorbent article on. Even though an undergarment of a boxer-shorts-type is illustrated in the figures of the present disclosure, it shall be readily understood that the undergarment may have any type of design, such as briefs or hipsters.

The undergarment 1 is further provided with a first landing zone 2 and a second landing zone 2', arranged in the crotch region 11 of the undergarment 1, on the inside of the undergarment. The landing zones are arranged to co-operate with attachment means of an absorbent article to be placed in the undergarment. The first landing zone 2 is positioned closer to the rear part 4 of the undergarment than the second landing zone 2'. Further, the second landing zone 2' is positioned closer to the front part 3 than the first landing zone 2. The landing zones 2, 2' indicate correct placement of an absorbent article in the undergarment.

Figure 1 further illustrates an exemplary absorbent article in shape of a pad 5 that may be placed in the undergarment 1. The pad comprises two attachment means 6, 6', arranged on one side of the pad: the side intended to face the undergarment, when the pad is placed in the undergarment. The attachment means 6, 6' comprises pressure sensitive adhesive. A pressure sensitive adhesive (PSA) is an adhesive that forms a bond with an adherend when pressure is applied to marry the adhesive with the adherend. Examples of pressure sensitive adhesives are D6312 from H.B. Fuller, DFCool600 from Henkel and H20028 from Bostik.

The attachment means 6, 6' covers a defined area of the side of the pad intended to face the undergarment. The attachment means may be an adhesive strip. The adhesive strip is preferably covered by a protection paper before it is taken in use. The protection paper is to be removed from the absorbent article before the absorbent article is placed in the undergarment. A first attachment means 6 is placed at a rear portion of the pad and a second attachment means 6' is placed at a front portion of the pad, in a longitudinal direction of the pad. The front portion of the pad is intended to be placed at the front part 3 of the undergarment and the rear portion of the pad is intended to be placed at the rear part 4 of the undergarment.

The landing zones 2, 2' of the undergarment 1 are arranged to cooperate with the attachment means 6 of the absorbent article 5. The landing zones 2, 2' are positioned in the undergarment to indicate correct placement of the attachment means 6, 6' of an absorbent article 5 in the undergarment 1. The landing zones indicate the shape of the attachment means of the absorbent article. When a user is about to place the pad 5 inside the undergarment 1, the user places the pad such that the landing zones 2, 2' cover the attachment means 6, 6' as illustrated by the arrows of figure 1.

The landing zones 2, 2' may have any shape or size or be placed essentially anywhere on the undergarment, as long as the landing zones match a correct placement of an attachment means of a pad to be placed in the undergarment.

The landing zones of fig. 1 may be embossed or printed on the undergarment.

Figure 2 shows an alternative undergarment having only one landing zone 2c, where the landing zone 2c corresponds to correct placement of a sole attachment means 6c of a different type of pad 5c.

According to the invention, the landing zones 2, 2', 2c each comprises a material having an affinity for pressure sensitive adhesive, for cooperating with attachment means 6, 6', 6c of an absorbent article, which attachment means comprises pressure sensitive adhesive. The material having an affinity for pressure sensitive adhesive may be e.g. a plastic film made of a plastic such as polyethylene, polypropene or polyvinyl chloride. The material having an affinity for pressure sensitive adhesive may further be a woven natural material, such as cotton, or a woven synthetic material, such as nylon, or a mixture of woven natural material and woven synthetic material.

Further, the landing zone may be accomplished by textile printing onto the material of the undergarment. In textile printing, ink paste is applied to the material of the undergarment in the area of the undergarment where the landing zone is to be positioned. In textile printing it may be important that the ink paste does not flow out, but have distinct contours. For that reason, there is often a thickening agent in the ink paste, either a natural thickening agent, such as starch or alginate or a synthetic thickening agent. After the ink paste has been applied to the undergarment and dried, the ink paste need to penetrate into fibers of the undergarment and bond to the fiber molecules, which may be performed by steaming or heat treatment.

According to another embodiment, the landing zone is a piece of material that is arranged separately to the undergarment, e.g. by being sewn or glued to the undergarment. The piece of material is selected such that it has an affinity for pressure sensitive adhesive.

The undergarment is preferably made of a woven material, a natural or synthetic woven material, or a mixture of a natural and synthetic material. The undergarment is preferably made of a material that can be stretched. Thereby, the undergarment will fit persons of different sizes. Although it may be necessary to have a number of different sizes of undergarment, the number of sizes may be limited if a high elasticity of material is achieved. The material of the undergarment may e.g. be made of a mixture of polyester and elastane. In an example the material of the undergarment is made of 87 % polyester and 13 % elastane.

At the same time as the undergarment has a high stretchability, or elasticity, the absorbent pad to be placed in the undergarment has a low elasticity. To avoid that the absorbent article is loosening from the undergarment when it is stretched, it is necessary to adapt the elasticity of the landing zone of the undergarment to the elasticity of the attachment means of the pad. For this reason, the landing zone is arranged to have an elasticity that is less than at least 30 % of the elasticity of a part of the undergarment arranged within a radius of 3 cm from the landing zone, preferably less than 20 %, more preferably less than 10 %, most preferably less than 5 %. This could be arranged by attaching the landing zone as a separate piece of material to the undergarment, e.g. by sewing the landing zone to the undergarment. Such a separate piece of material then needs to have an elasticity value less than at least 30 % of the elasticity value of the undergarment.

Further, when the landing zone and the attachment means have been joined together, the adhesive strength between the landing zone and the attachment means of the absorbent article is adapted to be 0.5 - 4 Newton, preferably 1.5 - 2 N, measured on an adhesive width of 45 mm. The adhesive strength may be manipulated by using different materials or by coating the landing zone with a coating agent, e.g. a release agent such as silicone, giving such an adhesive strength.

The adhesive strength, or the peel strength, between the landing zone and the attachment means of an absorbent article may be tested by the test described below.

Cut out an adhesive area from an absorbent pad to be used together with the undergarment. Place the adhesive area on a table with the adhesive/releasepaper side up, possibly after having grasped the topsheet and core together, peeled them away from the rest of the pad and scraped away any unattached fluff fibers clinging to the backsheet. The width of the adhesive area should be 45 mm. Cut out an area covering the landing zone from the undergarment. The area is preferably slightly larger than the adhesive area, e.g. 5-10 mm wider than the width of the adhesive area. Remove the releasepaper from the adhesive without touching the pressure sensitive adhesive glue (PSA glue). Make sure that the test sample is smooth and free from wrinkles. Put the landing zone of the undergarment on top of the PSA glue. Make sure no air bubbles are formed between the landing zone and the PSA-glue. Use a compression roller and move it slowly (3 m/min) to the front and then back again over the test sample. Fasten the test sample in a tensile tester machine, a first end of the landing zone in a first holder and a second end of the adhesive area in a second holder in such a way that the test will be made in a 180° angle. So it is possible to observe if anything happens with the PSA glue stripe. Thereafter, the machine tears off the landing zone from the adhesive area, the landing zone and the adhesive area are pulled in opposite directions. The machine measures the peel force and reports the peel force in Newton.

In general shall at least three samples be tested for each type of product, and a mean value of the three tests be taken as the correct value of the peel force. The equipment used in the test is a tensile tester machine called Instron; speed 100 mm/min, program 40, and a compression roller covered with rubber 45 mm wide and a total weight of 3,5 kg. All material shall be conditioned in 23 ± 1°C and 50 ± 2 % RH for at least 24 hours.

According to an embodiment of the invention, a surface area of the landing zone is arranged to be 10-30 % larger, preferably 15-25 % larger than a corresponding surface area of the attachment means of the absorbent article. By surface area of the landing zone and corresponding surface area of the attachment means are meant the areas of the landing zone and the attachment means that are to be joined to each other when the absorbent article is positioned in the undergarment, i.e. when the absorbent article is to be used in the undergarment.

The landing zones may alternatively (not shown) be placed at side parts of the crotch region.

There could be different landing zones in the undergarment adapted for male and for female users, respectively. For example, a female landing zone could have one colour or pattern and a male landing zone another colour or pattern.

The landing zone is advantageously such that it may withstand repeated washing of the undergarment, and the wear from repeated changing of the article. The landing zone is further preferably such that it is discrete, i.e. not visible from the outside of the undergarment.

Further, each landing zone may be divided into a number of sub-landing zones. I.e. in fig. 1 the first landing zone 2 may be divided into at least two sub-landing zones and the second landing zone 2' may be divided into at least two sub-landing zones. The sub-landing zones may for example be arranged as parallel stripes.

## Claims

1. An undergarment (1) for use with an absorbent article (5), wherein the undergarment is provided with at least one landing zone (2, 2') for cooperating with attachment means (6, 6') of the absorbent article, the at least one landing zone (2, 2') indicating placement of the attachment means of the absorbent article in the undergarment, **characterized in that** the landing zone (2, 2') comprises a material having an affinity for pressure sensitive adhesive.

2. The undergarment of claim 1, wherein the landing zone (2, 2') of the undergarment only has an affinity for pressure sensitive adhesive.

3. The undergarment of claim 1 or 2, wherein the landing zone (2, 2') is a piece of material that is arranged separately to the undergarment (1), e.g. by being sewn or glued to the undergarment.

4. The undergarment of any of claims 1-3, wherein the material of the landing zone (2, 2') is a plastic film, made of a plastic such as polyethylene, polypropene or polyvinyl chloride.

5. The undergarment of any of claims 1-3, wherein the material of the landing zone (2, 2') is a woven natural material, such as cotton, or a woven synthetic material, such as nylon.

6. The undergarment of any of claims 1-3, wherein the material of the landing zone (2, 2') is a mixture of a woven synthetic material and a woven natural material.

7. The undergarment of any of claims 1-3, wherein the material of the landing zone (2, 2') is ink paste, and wherein the ink paste has been applied to the undergarment.

8. The undergarment of any of the preceding claims, wherein after the landing zone (2, 2') and the attachment means (6, 6') of the absorbent article has been joined together, the adhesive strength between the landing zone and the attachment means of the absorbent article is 0.5 - 4 Newton, preferably 1.0 - 2.5 Newton, most preferably 1.5 - 2.0 Newton, measured on an adhesive width of 45 mm.

9. The undergarment of claim 8, wherein the landing zone (2, 2') is coated with a release agent.

10. The undergarment of claim 9, wherein the release agent is silicone.

11. The undergarment of any of the preceding claims, wherein the landing zone (2, 2') has an elasticity that is less than 30 % of the elasticity of a part of the undergarment arranged within a radius of 3 cm from the landing zone, preferably less than 20 %, more preferably less than 10 %, most preferably less than 5 %.

12. The undergarment of any of the preceding claims, wherein the position of the landing zone (2, 2') in the undergarment (1) corresponds to the position of the attachment means of an absorbent article, such that when the landing zone is joined to the attachment means of an absorbent article, the article is correctly placed in the undergarment.

13. The undergarment of any of the preceding claims, wherein a surface area of the landing zone (2, 2') is arranged to be 10-30 % larger, preferably 15-25 % larger, than a corresponding surface area of the attachment means of an absorbent article to be used with the undergarment.

14. The undergarment of any of the preceding claims, wherein the landing zone (2, 2') comprises two or more separate sub-landing zones.

15. A kit of parts comprising an undergarment (1) according to any of the preceding claims and at least one absorbent article (5; 5c) provided with attachment means (6, 6'; 6c) of a pressure sensitive adhesive for attaching the absorbent article to the undergarment.
